(19) Europäisches Patentamt
European Patent Office
Office européen des brevets

(11) Veröffentlichungsnummer: **0 158 225 B1**

(12) **EUROPÄISCHE PATENTSCHRIFT**

(45) Veröffentlichungstag der Patentschrift: **11.03.92**

(51) Int. Cl.[5]: **C12Q 1/34**

(21) Anmeldenummer: **85103672.3**

(22) Anmeldetag: **27.03.85**

# (54) Analysenverfahren und Mittel zum Nachweis esterolytischer und/oder proteolytischer Enzyme.

(30) Priorität: **06.04.84 DE 3413118**

(43) Veröffentlichungstag der Anmeldung:
**16.10.85 Patentblatt 85/42**

(45) Bekanntmachung des Hinweises auf die Patenterteilung:
**11.03.92 Patentblatt 92/11**

(84) Benannte Vertragsstaaten:
**AT BE CH DE FR GB IT LI LU NL SE**

(56) Entgegenhaltungen:
**EP-A- 0 014 252**
**EP-A- 0 034 323**
**EP-A- 0 039 880**
**EP-A- 0 094 554**
**EP-A- 0 133 329**
**EP-A- 0 134 291**
**FR-A- 2 190 277**

**CHEMICAL ABSTRACTS, Band 94, Nr. 19, 11. Mai 1981, Seite 279, Zusammenfassung Nr. 152503v, Columbus, Ohio, US; V. RAMESH et al.: "Studies on the kinetics and activation of soluble and immobilized sweet potato beta-amylase", & J. MOL. CATAL. 1981, 10(3), 341-55**

(73) Patentinhaber: **MILES INC.**
**1127 Myrtle Street**
**Elkhart Indiana 46514(US)**

(72) Erfinder: **Schnabel, Eugen, Dr.**
**Schimmelweg 6**
**W-5600 Wuppertal 11(DE)**
Erfinder: **Skjold, Christopher**
**1602 Victoria Drive**
**Elkhart Indiana 46514(US)**
Erfinder: **Travis, James, Prof.**
**Featherwood Hollow Road**
**Athens Georgia 30601(US)**

(74) Vertreter: **Adrian, Albert, Dr. et al**
**c/o BAYER AG Konzernverwaltung RP Patentabteilung**
**W-5090 Leverkusen 1, Bayerwerk(DE)**

EP 0 158 225 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS, Band 93, Nr. 15, 13. Oktober 1980, Seite 303, Zusammenfassung Nr. 145337v, Columbus, Ohio, US; J.P. BENEDETTO et al.: "Effects of low molecular weight basic proteins on the phosphohydrolase and phosphotransferase activities of microsomal glucose-6-phosphotase in adult monkey hepatocytes", & BIOCHIM. BIOPHYS. ACTA 1980, 614(2), 400-6

CHEMICAL ABSTRACTS, Band 95, Nr. 11, 14. September 1981, Seiten 385-386, Zusammenfassung Nr. 94612n, Columbus, Ohio, US; K.D. MILLER: "Nonenzymic control of prothrombin activation", & ANN. N.Y. ACAD. SCI. 1981, 370(CONTRIB. HEMOSTASIS), 336-47

CHEMICAL ABSTRACTS, Band 91, Nr. 9, 27. August 1979, Seite 263, Zusammenfassung Nr. 70776k, Columbus, Ohio, US; M. YAMAMOTO et al.: "A hepatic soluble cyclic nucleotide-independent protein kinase. Stimulation by basic polypeptides", & J. BIOL. CHEM. 1979, 254(12), 5049-52

**Beschreibung**

Die vorliegende Erfindung betrifft Mittel für den analytischen Nachweis von esterolytischen und/oder proteolytischen Enzymen, z.B. in Körperflüssigkeiten, wobei die Ester in geeigneter Weise in Testmitteln, insbesondere Teststreifen, inkorporiert sind. Die erfindungsgemäßen Mittel enthalten neben chromogenen Enzymsubstraten (Aminosäure- oder Peptidestern von geeigneten Phenolen) sowie gegebenenfalls mit den Phenolen unter Farbbildung kuppelnden Diazoniumsalzen noch besondere Salze als Beschleuniger für die enzymatische Spaltung der Aminosäure- bzw. Peptidester. Bevorzugt werden die Mittel für den Nachweis von Leukozyten, insbesondere im Urin, eingesetzt.

In der Diagnostik der Erkrankungen der Nieren und des Urogenitaltraktes hat der Nachweis von Leukozyten in Körperflüssigkeiten, insbesondere im Urin, große Bedeutung. Ursprünglich erfolgte dieser Nachweis durch Auszählen der Leukozyten im nicht zentrifugierten Harn oder im Harnsediment. Mit beiden Methoden können nur intakte Leukozyten erfaßt werden. Es ist jedoch bekannt, daß die Geschwindigkeit der Leukozyten-Lyse je nach Harnmilieu starken Schwankungen unterworfen ist; so beträgt z.B. in stark alkalischen Harnen die Leukozyten-Halbwertszeit nur 60 Minuten. Dies führt dazu, daß zu niedrige Leukozytenzahlen festgestellt werden. Von diesem Lyse-Fehler abgesehen, liefert die quantitative mikroskopische Bestimmung der Leukozyten im nicht zentrifugierten, homogenisierten Harn in der Zählkammer sehr genaue Werte. Trotzdem wird diese Methode in der Praxis nur selten angewandt, da sie mühevoll und zeitraubend ist und geschultes Personal voraussetzt.

Das in der medizinischen Praxis bevorzugte Verfahren für die Leukozytenbestimmungen im Harn war daher die sogenannte Gesichtsfeldmethode im Harnsediment. Hierzu mußte zunächst die Probe (Sediment) durch Zentrifugieren gewonnen werden. Dabei wurden jedoch auch andere Bestandteile des Harnes angereichert, die - wie z.B. Salze und Epithelzellen - die mikroskopische Auszählung der Leukozyten beträchtlich erschweren. Schwankender Sedimentgehalt, Inhomogenitäten des Sediments sowie unterschiedliche optische Ausstattung der Mikroskope führten zu relativ großen Fehlern (bis zu mehreren hundert Prozent) bei der Angabe der Leukozytenzahl.

Um diese Schwierigkeiten zu vermeiden, wurde bereits vielfach versucht, als Nachweisprinzip für Leukozyten in verschiedenen Körperflüssigkeiten enzymatische Reaktionen heranzuziehen, da die Leukozyten ein breitgefächertes Enzymspektrum besitzen.

So sind z.B. aus den deutschen Offenlegungsschriften 2 826 965 und 2 836 644 Mittel zum Nachweis von Leukozyten in Körperflüssigkeiten bekannt, bei denen die in Leukozyten vorhandene esterolytische und/oder proteolytische Aktivität für analytische Zwecke ausgenutzt wird. Als Substrate für die Leukozyten-Esterasen und/oder -Proteasen werden dabei Sulfonphthaleinester bzw. Azo-Farbstoffester verwendet. Die bei der enzymatischen Umsetzung freigesetzten Farbstoffe werden dann nach bekannten Methoden bestimmt. Die in diesen Publikationen beschriebenen Mittel sind jedoch für praktische Zwecke noch zu unempfindlich, da sie bei niedrigen Leukozyten-Konzentrationen zu lange Reaktionszeiten aufweisen.

Verschiedene Methoden für den Nachweis von Proteasen und Esterasen sind auch aus der histo- und cytochemischen Enzymologie bekannt (vgl. beispielsweise A.G.E. Pearse, Histochemistry, Theoretical and Applied, 3. Ed., Churchill Livingstone, Edinburgh-London-New York 1968). Zum Nachweis werden dabei im allgemeinen farblose oder schwach gefärbte Ester eingesetzt, die durch die Enzyme in eine farblose Säure und eine ebenfalls farblose Alkohol-(Phenol)-Komponente gespalten werden. Die Phenolkomponente wird dann in einer Folgereaktion zu farbigen Produkten umgesetzt, z.B. durch Kupplung mit Diazoniumsalzen oder durch Oxidation. F. Schmalzl und H. Braunsteiner beschreiben zum Beispiel in Klin. Wschr. 46, 642 (1968) einen spezifischen cytochemischen Leukozytenesterasenachweis mit Naphthol-AS-D-Chloracetat als Substrat und einem Diazoniumsalz, das mit dem freiwerdenden Naphthol eine farbige Azo-Verbindung bildet.

Für den schnellen und einfachen Nachweis von Leukozyten in Körperflüssigkeiten, wie z.B. im Harn, erwiesen sich Zweikomponenten-Systeme dieser Art jedoch als nicht geeignet, da sie viel zu unempfindlich sind: Proben mit 5000 Leukozyten/µl zeigen noch keine Reaktion.

In GB-A 1 128 371 und EP-A 12 957 wird die Verwendung von Indoxyl- und Thioindoxylestern als chromogenen Substraten für den Nachweis von hydrolytischen Enzymen in Körperflüssigkeiten beschrieben. Bei der enzymatischen Spaltung des Substrats entsteht freies Indoxyl, welches anschließend zum leicht nachweisbaren blauen Farbstoff Indigo oxidiert wird. Ein handelsüblicher Test auf Basis von EP-A 12 957 besteht aus einem Streifen Filterpapier, welches mit dem N-Tosyl-L-alanin-indoxylester imprägniert ist. Beim Eintauchen in eine Leukozyten enthaltende Urinprobe färbt sich der Teststreifen blau. Ein wesentlicher Nachteil dieses Produkts ist jedoch die lange Wartezeit (ca. 15 Minuten), bis die Endfärbung erreicht ist und der Test ausgewertet werden kann.

In EP-A 14 929 werden verschiedenartige Beschleuniger (Pyridinderivate; Imidazolderivate; Alkohole;

Metallkomplexe) für die enzymatische Spaltungsreaktion beschrieben. Nachteilig bleiben jedoch die relativ lange Zeit bis zur vollständigen Oxidation des Indoxyls und die geringe Empfindlichkeit des Tests (Nachweisgrenze: einige Tausend Leukozyten/µl). Gleiches trifft auch für die Verwendung der Ester von Leuko-Indoanilinen als Substrate für Leukozyten-Enzyme gemäß EP-A 34 323 zu.

EP-A 39 880 stellt eine Kombination der Substrate gemäß EP-A 12 957 bzw. 14 929 mit dem weiter oben diskutierten Nachweisprinzip der Kupplung mit Diazoniumsalzen dar. Es gelingt auf diese Weise zwar, die Erfassungsgrenzen für Leukozyten deutlich zu senken, jedoch wird die für die Praxisanwendung gewünschte Nachweisempfindlichkeit von 15 - 20 Leukozyten/µl noch nicht erreicht.

Aufgabe der vorliegenden Erfindung war es daher, neue Aktivatoren für esterspaltende Enzyme aufzufinden, die infolge einer Beschleunigung der enzymatischen Spaltung der Substrate durch die Leukozytenenzyme einen empfindlichen und rascheren Nachweis der Leukozyten im Harn ermöglichen. Diese Aufgabe wird durch den Zusatz von Salzen zu dem Reagenssystem gelöst.

Wie überraschenderweise gefunden wurde, wird die Spaltung der chromogenen Substrate durch Leukozytenenzyme durch Zusatz besonderer Salze erheblich beschleunigt, bzw. die Wirkung im Testsystem bereits vorhandener Aktivatoren potenziert.

Gegenstand der Erfindung sind Mittel zum Nachweis esterolytischer und/oder proteolytischer Enzyme, enthaltend

(a) einen Aminosäure- oder Peptidester eines Phenols als chromogenes Enzymsubstrat,

(b) eine die enzymatische Spaltung der Aminosäure- bzw. Peptidesterbindung von Komponente (a) beschleunigende Substanz, gegebenenfalls

(c) ein Diazoniumsalz, gegebenenfalls

(d) einen Puffer, sowie gegebenenfalls

(e) ein Trägermaterial und/oder übliche Zusatzstoffe,

welche dadurch gekennzeichnet sind, daß als beschleunigende Substanz, gegebenenfalls gemeinsam mit anderen Aktivatoren, ein Salz eines Kations der Gruppe $Li^+$, $Na^+$, $K^+$ oder $Mg^{++}$ und eines Anions der Gruppe Halogenid, Pseudohalogenid, Sulfat, Phosphat, Acetat, Trifluoracetat, Toluolsulfonat oder Succinat eingesetzt wird.

Gegenstand der Erfindung ist schließlich auch ein Verfahren zum Nachweis von esterolytischen und/oder proteolytischen Enzymen in flüssigen Proben, insbesondere Körperflüssigkeiten, welches dadurch gekennzeichnet ist, daß man die Probe mit dem erfindungsgemäßen Mittel in Kontakt bringt und die auftretende Farbreaktion bestimmt.

Beschleunigend wirkende Salze sind erfindungsgemäß Salze von ein- und zweiwertigen Kationen der Alkali- und Erdalkalimetalle $Li^+$, $Na^+$, $K^+$ und $Mg^{++}$. Die Anionen der aktivierenden Salze sind Halogenide und Pseudhalogenide, Sulfate und Phosphate sowie die Ionen organischer Säuren wie Essigsäure, Trifluoressigsäure, Toluolsulfonsäure und Bernsteinsäure.

Erfindungsgemäß liegen die Konzentrationen der Salze in den Testlösungen zwischen 0,05 M und 2M, bevorzugt zwischen 0,1 und 1 M.

In den für die Imprägnierung von Trägern (bei der unten beschriebnene Herstellung von Testvorrichtungen) verwendeten Formulierungen werden die Salze vorzugsweise in Mengen von 0,01 M bis 2 M, besonders bevorzugt von 0,1 bis 1 M gelöst.

Die erfindungsgemäß zu verwendenden Salze beschleunigen die enzymatische Spaltung der in den EP-A 7407, 8428, 12 557, 14 929, 34 323 und 39 880 beschriebenen Substrate durch die Leukozytenenzyme ebenso wie die Spaltung der bereits früher beschriebenen Substrate [G.Gomori, J. Histochem. Cytochem. 6 469 (1953); H. Löffler, Klin. Wochenschr. 39, 1120 (1961); L. Visser und E. Blout, Fed.Proc. 28, 407 (1969) sowie Biochim. Biophys. Acta 268, 257 (1972) und R.Sweetman und L. Ornstein, J. Histochem. Cytochem. 23, 327 (1974)].

Zu den bevorzugten chromogenen Substraten in den erfindungsgemäßen Mitteln gehören auch die in der parallelen Anmeldung EP.A 157 360 beschriebenen Verbindungen der allgemeinen Formel

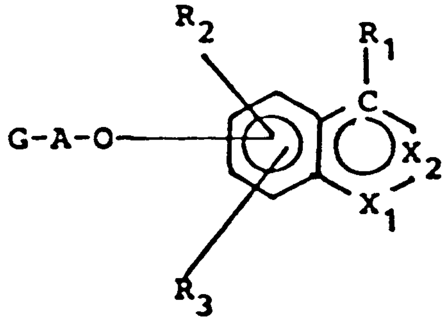

(II)

in welcher

- $X_1$ und $X_2$ gleich oder verschieden sind und Stickstoff oder Schwefel bedeuten, mit der Maßgabe, daß $X_1$ und $X_2$ nicht gleichzeitig für Schwefel stehen;
- $R_1$ für Wasserstoff oder eine gegebenenfalls verzweigte Alkylgruppe mit 1 bis 6 C-Atomen steht, die gegebenenfalls durch Halogen oder Hydroxy substituiert sein kann,
- $R_2$ und $R_3$ gleich oder verschieden sind und für Wasserstoff, $C_1$-$C_6$-Alkylgruppen, $C_1$-$C_6$-Alkoxygruppen, $C_1$-$C_6$-Acylgruppen, Halogen, Trifluormethyl, Nitro, $SO_3H$, Cyano, $C_1$-$C_8$-Acylaminogruppen, $C_1$-$C_6$-Dialkylaminogruppen oder $C_6$-$C_{10}$-Arylgruppen stehen, die ihrerseits wieder durch $C_1$-$C_6$-Alkylgruppen, $C_1$-$C_6$-Alkoxygruppen, Halogen, Cyano, Nitro, Trifluormethyl, $SO_3H$, $C_1$-$C_6$-Acylgruppen oder $C_1$-$C_6$-Dialkylaminogruppen substituiert sein können, oder $R_2$ und $R_3$ gemeinsam einen annellierten aromatischen Ring, vorzugsweise einen Benzolring, bilden, welcher seinerseits mit einem oder zwei Resten $R_2$ substituiert sein kann;
- A einen Aminosäure- oder Peptidrest bedeutet und
- G Wasserstoff oder vorzugsweise eine in der Peptidchemie übliche oder davon abgeleitete Stickstoffschutzgruppe darstellt.

Bevorzugte Verbindungen der allgemeinen Formel (II) sind solche, bei denen $X_1$ für Schwefel und $X_2$ für Stickstoff stehen. Bevorzugt sind weiterhin Verbindungen der Formel (II), in denen $R_1$ für Wasserstoff steht und solche, bei denen $R_2$ und $R_3$, die gleich oder verschieden sind, für Wasserstoff, $C_1$-$C_2$-Alkyl,$C_1$-$C_2$-Alkoxy, Halogen, $C_1$-$C_4$-Dialkylaminogruppen oder Benzolreste stehen.

Besonders bevorzugt steht bei den Verbindungen von Formel (II) der Esterrest in 5-Position.

Weitere erfindungsgemäß bevorzugte chromogene Substrate sind die in der parallelen Anmeldung EP-A 157 362 beschriebenen Verbindungen der allgemeinen Formel

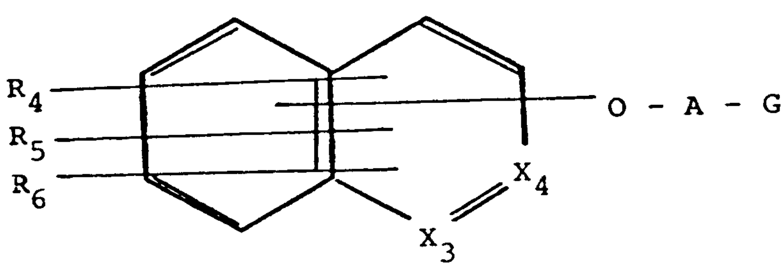

(III)

in welcher

- $X_3$ und $X_4$ für N oder CH stehen, mit der Maßgabe, daß jeweils entweder $X_3$ oder $X_4$ für N steht,
- $R_4$, $R_5$ und $R_6$ gleich oder verschieden sind und für Wasserstoff, $C_1$-$C_6$-Alkylgruppen, $C_1$-$C_6$-Alkoxygruppen, $C_1$-$C_6$-Acylgruppen, Halogen, Trifluormethyl, Nitro, $SO_3H$, Cyano, $C_1$-$C_8$-Acylaminogruppen, $C_1$-$C_6$-Dialkylaminogruppen oder $C_6$-$C_{10}$-Arylgruppen stehen, die ihrerseits wieder durch $C_1$-$C_6$-Alkylgruppen, $C_{1-6}$-Alkoxygruppen Halogen, Cyano, Nitro, Trifluormethyl, $SO_3H$, $C_1$-$C_6$-Acylgruppen oder $C_1$-$C_6$-Dialkylaminogruppen substituiert sein können, oder $R_5$ und $R_6$ gemeinsam einen annellierten aromatischen Ring, vorzugsweise einen Benzolring, bilden, welcher seinerseits mit einem oder zwei Resten $R_4$ substituiert sein kann;

und A und G die oben bei Formel (II) angegebene Bedeutung haben.

In den Verbindungen gemäß allgemeiner Formel (III) steht vorzugsweise $X_3$ für CH und $X_4$ für Stickstoff.

Bevorzugt bedeuten $R_4$, $R_5$ und $R_6$, die gleich oder verschieden sein können, für Wasserstoff, $C_1$-$C_4$-Alkyl, $C_1$-$C_4$-Alkoxy, Acylamino (wobei der Säurerest aliphatisch oder aromatisch mit 1 - 6 C-Atomen sein kann), $C_1$-$C_4$-Dialkylamino, Nitro, Cyano, Halogen sowie Aryl, das gegebenenfalls substituiert ist durch $C_1$-$C_4$-Alkyl, $C_1$-$C_4$-Alkoxy oder Halogen. Besonders bevorzugt sind $R_4$, $R_5$ und $R_6$ Wasserstoff, $C_1$-$C_4$-Alkyl, Phenyl oder Halogen oder $R_5$ und $R_6$ bilden zusammen einen annellierten Benzolring.

Als chromogene Substrate für die erfindungsgemäßen Mittel eignen sich darüber hinaus auch Verbindungen der allgemeinen Formel

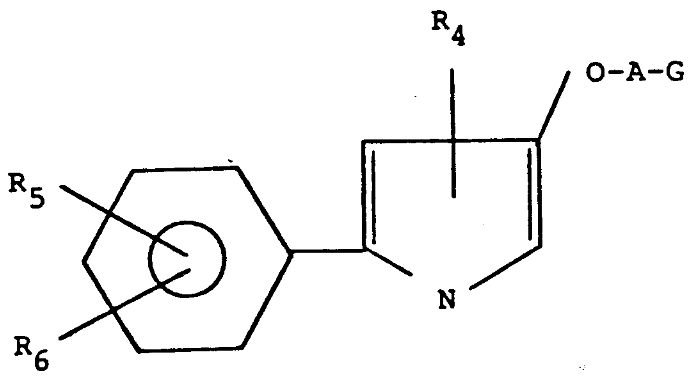

(IV)

wobei $R_4$, $R_5$, $R_6$, A und G die oben bei Formel (III) angegebene Bedeutung haben.

In den allgemeinen Formeln (II), (III) und (IV) steht G - A - bevorzugt für einen Rest der allgemeinen Formel

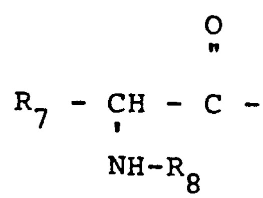

in welcher

$R_7$ für Wasserstoff oder einen gegebenenfalls verzweigten Alkyl-, Cycloalkyl- oder Arylrest mit 1-15 C-Atomen, bevorzugt 1-9 C-Atomen, steht, welcher gegebenenfalls durch eine Hydroxy-, eine Mercapto- oder eine Carboxylgruppe substituiert ist, und

$R_8$ Wasserstoff oder vorzugsweise -CO-Alkyl, -CO-Aralkyl, -CO-Aryl, $-SO_2$-Alkyl oder $-SO_2$-Aryl darstellt, wobei Alkylreste geradkettig oder verzweigt mit 1-9 C-Atomen, bevorzugt 1-6 C-Atomen sind und Arylreste bevorzugt Benzolringe, die gegebenenfalls durch $C_1$-$C_4$-Alkylgruppen, $C_1$-$C_4$-Alkoxygruppen oder Halogen substituiert sind, darstellen.

Besonders bevorzugt steht G-A- für einen mit einer üblichen Stickstoffschutzgruppe versehenen Rest einer natürlichen Aminosäure oder eines Peptids aus 2 bis 8 solcher Aminosäuren.

Die Aminosäurereste können dabei in ihrer L- oder D-Form oder auch in ihrer racemischen Form vorliegen. Besonders bevorzugt sind die Reste von Glycin, Alanin, Valin, Leucin, Isoleucin, Phenylalanin und Tyrosin, wobei jeweils die L-Form besonders bevorzugt ist. Gegebenenfalls vorhandene freie Hydroxygruppen können acyliert, vorzugsweise acetyliert, sein.

Unter einem Peptidrest in der Definition von A sind z.B. Di-, Tri-, Tetra- und Pentapeptide, vorzugsweise Di- und Tripeptide, zu verstehen, wobei als Aminosäure-Komponenten vorzugsweise die oben erwähnten Aminosäuren in Betracht kommen.

Die Substrate der allgemeinen Formeln (II), (III) und (IV) werden erhalten, indem man die entsprechenden Phenole mit Aminosäuren bzw. Peptiden der allgemeinen Formel

G - A - OH

in welcher G und A die oben angegebene Bedeutung haben, bzw. geeigneten reaktiven Derivaten davon nach in der Peptidchemie üblichen Methoden umsetzt.

Geeignete reaktive Derivate sind z. B. die Säurechloride und die bei der Peptidsynthese üblicherweise verwendeten Mischanhydride, z. B. mit Chlorameisensäureethylester, oder Aktivester wie z. B. Pentachlorphenylester oder N-Hydroxybenztriazolester.

Die erfindungsgemäßen Mittel enthalten vorzugsweise als mit den (bei der enzymatischen Spaltung freigesetzten) Phenolen reagierende Farbbildner Diazoniumsalze der allgemeinen Formel

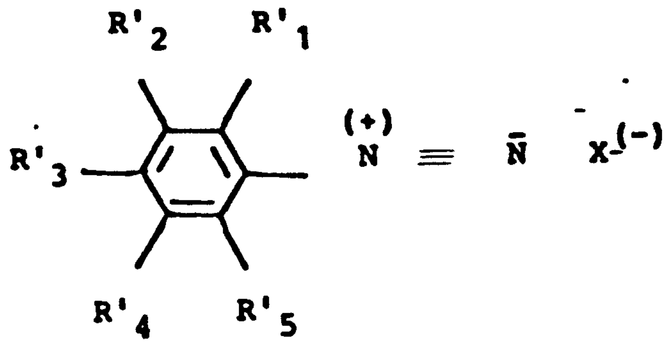

(V)

in der

| | |
|---|---|
| $R'_1$ | eine niedere Alkyl-, eine niedere Alkoxy-, eine niedere Alkylmercapto-, eine Hydroxy-, Nitro-, Cyano-, Trifluormethyl-, $C_1$-$C_8$-Alkylsulfonamido-, Arylsulfonamido-, $C_1$-$C_8$-Alkylsulfon-, Arylsulfon-, Sulfonsäure-, Carbonsäure-, eine N-Morpholino-, eine N-Thiomorpholino-, eine N-Pyrrolidino-, eine gegebenenfalls N'-alkylierte N-Piperazino-, eine N-Piperidino-Gruppe, Halogen oder Wasserstoff, |
| $R'_3$ | eine niedere Alkyl-, eine niedere Alkoxy-, eine Aryloxy-, eine niedere Alkylmercapto-, Alkylamino-, Dialkylamino-, eine Hydroxy-, Nitro-, Cyano-, $C_1$-$C_8$-Alkylsulfonamido-, Arylsulfonamido-, $C_1$-$C_8$-Alkylsulfon-, Arylsulfon-, Sulfonsäure-, Carbonsäure-, eine N-Morpholino-, N-Thio-morpholino-, N-Pyrrolidino-, eine gegebenenfalls N'-alkylierte N-Piperazino-, N-Piperidino-, Phenylamino-, eine gegebenenfalls mit einem niederen Alkyl- oder niederen Alkoxy-Rest substituierte Phenyl-Gruppe, Halogen oder Wasserstoff, |
| $R'_2$, $R'_4$, $R'_5$, | die gleich oder verschieden sein können, jeweils eine niedere Alkyl-, eine niedere Alkoxy-, Nitro-, $C_1$-$C_8$-Alkylsulfonamido-, Arylsulfonamido-, $C_1$-$C_8$-Alkylsulfon-, Arylsulfon-, Sulfonsäure-, Carbonsäure-, eine niedere Alkylmercapto-Gruppe, Halogen oder Wasserstoff und |
| X | ein stabilisierendes Anion |

bedeuten, wobei jeweils 2 benachbarte Reste $R'_1$ bis $R'_5$ zu einem gegebenenfalls durch Halogen, eine $C_1$-$C_6$-Alkyl-, eine $C_1$-$C_6$-Alkoxy-, eine Nitro-, Sulfonsäure- oder Carbonsäuregruppe substituierten Benzolring ringgeschlossen sein können, so daß ein Diazoniumsalz der Naphthalinreihe entsteht.

Vorzugsweise stehen in der allgemeinen Formel (V)

| | |
|---|---|
| $R'_1$ | für $C_1$– bis $C_4$-Alkyl, $C_1$-$C_4$-Alkoxy, Hydroxy, Nitro, Halogen oder Wasserstoff, |
| $R'_3$ | für eine $C_1$-bis $C_4$-Alkyl-, $C_1$-$C_4$-Alkoxy-, Aryloxy-, $C_1$-$C_4$-Alkylamino-, $C_1$-$C_4$-Dialkylamino-, Nitro-, $C_1$-$C_4$-Alkyl-Sulfonamido-,Arylsulfonamido-, $C_1$-$C_4$-Alkylsulfon-, Arylsulfon-, N-Morpholino-, N-Pyrrolidino-, Phenylamino- oder Sulfonsäuregruppe oder Wasserstoff und |
| $R'_2$, $R'_4$, $R'_5$, | die gleich oder verschieden sein können, für $C_1$- bis $C_4$-Alkyl-, $C_1$- bis $C_4$-Alkoxy-, $C_1$- bis $C_4$-Alkylamino-,$C_1$- bis $C_4$-Dialkylamino-, Nitro-, $C_1$- bis $C_4$-Alkylsulfonamido-, Arylsulfonamido- oder Sulfonsäuregruppen, Halogen oder Wasserstoff. |

Jeweils 2 benachbarte Reste $R'_1$ bis $R'_5$ können dabei gegebenenfalls zu einem gegebenenfalls durch Halogen, eine $C_1$- bis $C_4$-Alkyl-, $C_1$- bis $C_4$-Alkoxy-, eine Nitro- oder Sulfonsäuregruppe substituierten Benzolring ringgeschlossen sein.

Im Rahmen der Formel (V) steht Aryl jeweils für einen gegebenenfalls durch Halogen, eine $C_1$-$C_4$-Alkyl- oder $C_1$-$C_4$-Alkoxygruppe substituierten aromatischen Rest mit 6 bis 12 C-Atomen, vorzugsweise 6 C-Atomen.

Die Diazoniumsalze der allgemeinen Formel (V) sind an sich bekannt oder können nach an sich bekannten Methoden synthetisiert werden (Houben-Weyl, Methoden der organischen Chemie, Band X/3).

Vorzugsweise enthalten die erfindungsgemäßen Mittel zum Nachweis proteolytischer Enzyme und insbesondere der Leukozyten-Enzyme ein geeignetes Puffersystem. Hierfür kommen z. B. Phosphat-, Borat-, Carbonat/Hydrogencarbonat-, Carbonat-, Barbiturat-, Tris-(hydroxymethyl)-aminomethan-(=Tris), 2-Amino-2-methyl-propandiol-1,3-(=Amediol)- oder Aminosäure-Puffer in Frage, wobei in der Regel pH-Wert und Kapazität so gewählt werden, daß sich in der Meßlösung bzw. auf dem Teststreifen ein pH-Wert von 6 - 10, vorzugsweise von 7 - 9, einstellt.

Vorzugsweise enthalten die erfindungsgemäßen Mittel neben den beschleunigend wirkenden Salzen noch weitere Aktivatoren, die zum Teil an sich bekannt sind. Beispiele geeigneter Aktivatoren sind die in EP-A 14 929 beschriebenen Pyridinderivate, Imidazolverbindungen, Metallkomplexe und insbesondere Alkohole, wobei n-Decanol und vor allem n-Undecanol besonders bevorzugt sind. Darüber hinaus seien als bisher noch nicht bekannte Beschleuniger der enzymatischen Spaltungsreaktionen basische Aminosäuren enthaltende Homo- und Co-Polyaminosäuren [E.Katchalski und M. Sela in: Advances of Protein Chemistry 13, 243 - 492 (1958); C.B. Anfinsen, M.L. Anson, J.T. Edsall und K. Bailey (Hrsg.); Academic Press Inc. Publishers, New York, N.Y.] sowie sequentielle Polyaminosäuren genannt, wie sie in einer parallelen Patentanmeldung beschrieben werden. Als basische Aminosäuren kommen solche Aminosäuren in Frage, die in den Seitenketten Amino- oder Guanidinogruppen tragen. Es sind dies insbesondere Lysin und Ornithin sowie Arginin aber auch nicht in natürlichen Proteinen vorkommende basische Aminosäuren wie beispielsweise Diaminobuttersäure, Diaminopropionsäure oder Diaminopimelinsäure. Die in den Polyaminosäuren enthaltenen Aminosäuren können racemisch oder optisch aktiv in der D- oder L-Form vorliegen. Die Molekulargewichte der Polyaminosäuren liegen zwischen 1000 und 2 000 000, vorzugsweise jedoch zwischen 5000 und 500 000. Die Gehalte an basischen Aminosäuren betragen zwischen 5 und 100 Molprozent, vorzugsweise 20 bis 100 Molprozent, bezogen auf Polyaminosäure.

Die erfindungsgemäßen Mittel können auch an sich bekannte Detergentien enthalten, da hierdurch eine homogenere Farbverteilung und eine intensivere Färbung erzielt werden können. In Frage kommen sowohl kationenaktive als auch anionenaktive Detergentien aber auch amphotere nichtionogene Detergentien. Als Beispiele hierfür seien Benzyldimethyl-tetradecyl-ammoniumchlorid, Natrium-dodecylsulfat, Zephirol, Polyvinylpyrrolidon, Heparinoid sowie die bereits als Aktivatoren aufgeführten Polyaminosäuren und Sequenzpolymeren genannt. Gegebenenfalls können auch Mischungen von zwei oder mehr der oben aufgeführten Detergentien verwendet werden.

Vorzugsweise sind bei den erfindungsgemäßen Mitteln die verschiedenen oben beschriebenen Reagenzien in einem inerten Trägermaterial der an sich bekannten Art inkorporiert, wobei als Trägermatrix besonders bevorzugt poröse Materialien wie insbesondere Filterpapier, aber auch Kunststoffmembranen, Glasfasermatten (US-PS 3 846 247), poröse Keramikstreifen, Kunstfaservliese, schwammartige Materialien (US-PS 3 552 928), Filz, Textilien, Holz, Cellulose oder auch Silicagel in Frage kommen.

Die genannten Trägermaterialien werden zu diesem Zweck mit einer Lösung der oben beschriebenen Reagentien in einem geeigneten leicht entfernbaren Lösungsmittel, z.B. Wasser, Methanol, Ethanol, Aceton, DMF oder DMSO imprägniert. Vorzugsweise geschieht dies in zwei getrennten Schritten: Zunächst wird mit einer wäßrigen Lösung imprägniert, die den Puffer und andere wasserlösliche Zusatzstoffe enthält. Danach wird mit einer Lösung der chromogenen Enzym-Substrate der allgemeinen Formel (V) und Aktivatoren imprägniert. Die Imprägnierung kann jedoch auch in einer anderen Reihenfolge bzw. mit einer anderen Zusammensetzung der beiden Imprägnierlösungen durchgeführt werden. Vorzugsweise enthält die Imprägnierlösung oder die zu untersuchende Flüssigkeit das chromogene Substrat und das Diazoniumsalz jeweils in einer Konzentration von $10^{-4}$ bis $10^{-1}$ mol/l, insbesondere $10^{-3}$ bis $10^{-2}$ mol/l, und das Salz in einer Konzentration von O,O1 M bis 2 M, insbesondere von O,1 M bis 1 M.

Bei Verwendung von Filterpapier als Matrix können die fertigen Testpapiere als solche verwendet werden oder in an sich bekannter Weise an Griffen angeklebt oder vorzugsweise zwischen Kunststoffen und feinmaschigen Netzwerken z.B. gemäß DE-OS 2 118 455 eingesiegelt werden.

Zur Herstellung filmbeschichteter Teststreifen werden vorzugsweise sämtliche Reagenzien in die Lösung oder Dispersion einer filmbildenden Substanz, wie z.B. Polyvinylester oder Polyamid, eingetragen und homogen vermischt. Das Gemisch wird in dünner Schicht auf einen Kunststoffträger gestrichen und getrocknet. Die so hergestellten filmbeschichteten Teststreifen werden nach dem Trocknen geschnitten und können als solche verwendet oder in an sich bekannter Weise an Griffen angeklebt werden oder z.B. zwischen Kunststoffen und feinmaschigen Netzwerken gemäß DE-OS 2 118 455 eingesiegelt werden.

Ein erfindungsgemäßes diagnostisches Mittel zum Nachweis esterolytischer und/oder proteolytischer Enzyme, insbesondere der Leukozyten-Enzyme, in Form von Pulvermischungen oder Reagenztabletten läßt sich herstellen, indem die oben angeführten Bestandteile des Testmittels mit üblichen galenischen Zusatzstoffen versetzt und granuliert werden. Zusatzstoffe dieser Art sind z.B. Kohlenhydrate, wie z.B. Mono-, Oligo- oder Polysaccharide, oder Zuckeralkohole, wie z.B. Mannit, Sorbit oder Xylit, oder andere lösliche inerte Verbindungen, wie Polyethylenglykole oder Polyvinylpyrrolidon. Die Pulvermischungen oder Reagenztabletten weisen z.B. ein Endgewicht von ungefähr 50 - 200 mg, vorzugsweise 50 - 80 mg, auf.

Zur Herstellung von Lyophilisaten im Gesamtgewicht von jeweils etwa 5 - 20 mg, vorzugsweise etwa 10 mg, wird eine Lösung gefriergetrocknet, die neben sämtlichen für den Test benötigten Reagenzien übliche Gerüstbildner, wie z.B. Polyvinylpyrrolidon, und evtl. weitere Füllstoffe, wie z.B. Mannit, Sorbit oder Xylit, enthält.

Ein erfindungsgemäßes diagnostisches Mittel in Form einer Lösung enthält vorzugsweise sämtliche für den Test benötigten Reagenzien. Als Lösungsmittel kommen Wasser oder Gemische von Wasser mit einem wasserlöslichen organischen Lösungsmittel, wie z.B. Methanol, Ethanol, Aceton oder Dimethylformamid, in Frage. Aus Haltbarkeitsgründen kann es vorteilhaft sein, die für den Test benötigten Reagenzien auf zwei oder mehr Lösungen zu verteilen, die erst bei der eigentlichen Untersuchung zusammengegeben werden.

Die so hergestellten diagnostischen Mittel ermöglichen es, nach Eintauchen in die zu untersuchende oder nach Zugabe zur betreffenden Körperflüssigkeit die Anwesenheit esterolytischer und/oder proteolytischer Enzyme, insbesondere der Leukozyten-Enzyme, rasch und einfach über eine Farbbildung nachzuweisen, die visuell oder photometrisch, z.B. remissions-photometrisch oder in der Küvette, gemessen werden kann. Da die Aktivität der Leukozyten-Enzyme pro Zelle als eine im wesentlichen konstante Größe angesehen werden kann, läßt sich aus der Intensität der Farbbildung die Leukozytenkonzentration der untersuchten Körperflüssigkeit ermitteln. Dabei werden mit dem erfindungsgemäßen diagnostischen Mittel sowohl intakte als auch lysierte Leukozyten erfaßt, da die Aktivität der Leukozyten-Enzyme auch nach der Lyse der Leukozyten voll erhalten bleibt. Ein Lysefehler tritt folglich nicht auf.

Die nachfolgenden Beispiele dienen zur Erläuterung der vorliegenden Erfindung. Wenn nicht anders vermerkt, sind Mengenangaben als Gewichtsteile oder Gewichtsprozente zu verstehen.

Allgemeine Vorgangsweise

Zu 2.25 ml des betreffenden, das Salz enthaltenden Puffers fügte man je nach Substrat 50 - 250 µl N-Methylpyrrolidon als Lösungsvermittler und ergänzte das Volumen der Lösung mit Puffer auf 2.5 ml. Dann wurden gegebenenfalls 5 µl einer Lösung von 5-100 mg zusätzlicher Aktivatoren in 1 ml N-Methylpyrrolidon sowie 5 µl einer Lösung von 2 mg Natriumdodecylsulfat (SDS) bzw. 4 mg der anderen Detergentien in 1 ml Wasser oder N-Methylpyrrolidon zu der Reaktionsmischung zugegeben. Nach gutem Durchmischen wurden 5 µl einer $10^{-1}$ molaren Substratlösung in N-Methylpyrrolidon, Dimethylformamid oder Dimethylsulfoxid in die Reaktionslösung gegeben und nach der Zugabe der Leukozytensuspension der Extinktionsanstieg bei der angegebenen Wellenlänge kontinuierlich verfolgt. Die Spontanhydrolyse des Substrats wird in einem Parallelansatz ohne Leukozytenzugabe anhand des Extinktionsanstiegs bestimmt.

Für die Bestimmung der Umsetzungsgeschwindigkeit wird der in den Enzymreaktion erhaltene Extinktionsanstieg um den für die Spontanhydrolyse ermittelten Betrag vermindert. Absolutwerte für die Substratspaltung (Mol. Min.$^{-1}$) können aus den Extinktionsdifferenzen mit Hilfe der molaren Extinktionskoeffizienten errechnet werden.

Beispiel 1

Filtrierpapier (z. B. Eaton und Dikeman 205) wird nacheinander mit den folgenden Lösungen imprägniert und dann bei 60 °C getrocknet.

Lösung 1:

O.1 M Tris-(hydroxymethyl)-aminomethan-Salzsäure-Puffer (pH 8.8), enthaltend O,25 M Natriumchlorid und 3 % Polyvinylpyrrolidon.

Lösung 2:

7.5 · $10^{-3}$ Mol/l N-Tosyl-L-alanin-5-hydroxy-1.2-benzisothiazolylester, $10^{-2}$ Mol/l 2,4-Dimethoxybenzoldiazonium-tetrafluoroborat und 3.5 g n-Undecanol/l in wasserfreiem Aceton.

Man erhält ein schwach gelb gefärbtes Testpapier, das sich beim Eintauchen in leukozytenhaltige Urine rotbraun verfärbt.

Beispiel 2

Filterpapier (z. B. Eaton und Dikeman 205) wird nacheinander mit den folgenden Lösungen imprägniert

und dann bei 60 ° C getrocknet.

Lösung 1:

O.1 M Borat-Puffer (pH = 9.0), enthaltend 3 % Polyvinylpyrrolidon und O.25 Mol Natriumchlorid/l.

Lösung 2:

$5 \cdot 10^{-3}$ Mol/l N-Tosyl-L-alanin-3-hydroxy-5-phenyl-pyrrolester.

Lösung 3:

$10^{-2}$ Mol/l 2-Hydroxy-4-sulfo-naphthyldiazonium-tetrafluoroborat und 3.5 g Decanol/l in wasserfreiem Aceton.

Man erhält ein schwach gelb gefärbtes Testpapier, das sich beim Eintauchen in leukozytenhaltige Urine rot bis violett verfärbt.

Beispiel 3

Einfluß der Salzkonzentration auf die Spaltung von N-Tosyl-L-alanin-indoxylester in O.1 M Boratpuffer (pH 8.8) bzw. 0.1 M Tris-(hydroxymethyl)-aminomethan-Puffer (pH 8.8) mit Leukozyten unter Zusatz von 10 µg SDS und 125 µg n-Decanol/Testansatz.

Tabelle 1

| Puffer | Salz | Molarität | relative Spaltungsgeschwindigkeit |
|---|---|---|---|
| Borat | LiCl | 0,25 | 1.25 |
| | NaBr | " | 1.15 |
| | NaCl | " | 1.3 |
| | NaOCN | " | 1.38 |
| | Na-Phosphat | 0,2 | 3.2 |
| | NaSCN | 0,25 | 1.42 |
| Tris | - | - | 1 |
| | NaCl | 0.1 | 1.0 |
| | " | 0.25 | 1.5 |
| | " | 0.5 | 2.3 |
| | " | 1.0 | 1.75 |
| | Na-Phosphat | 0.2 | 3.25 |
| | Natrium-toluolsulfonat | 0.1 | 1.45 |
| | " | 0.25 | 1.6 |
| | " | 0.5 | 1.8 |
| | Natrium-trifluoracetat | 0.1 | 1.1 |
| | " | 0.25 | 2.6 |
| | " | 0.5 | 2.3 |
| | Magnesiumchlorid | 0.25 | 1.9 |

Beispiel 4

Einfluß der Salzkonzentration auf die Spaltung von N-Tosyl-L-alanin-indoxylester in 0.1 M Tris-(hydroxymethyl)-aminomethan-Salzsäurepuffer (pH 8.8) mit Leukozyten, in Gegenwart von 10 µg SDS und 125 µg n-Decanol/Test.
(Bestimmung der Spaltung bei 360 nm).

Tabelle 2

| Salz | Molarität | relative Spaltungs geschwindigkeit |
|---|---|---|
| - | - | 1 |
| Natriumacetat | 0.25 | 2.5 |
| " | 0.5 | 2.8 |
| | 1.0 | 4.2 |
| Natriumsuccinat | 0.1 | 3.9 |
| " | 0.25 | 4.0 |
| " | 0.5 | 3.7 |
| " | 1.0 | 1.95 |

Beispiel 5

Einfluß der Salzkonzentration auf die Spaltung von N-Tosyl-L-alanin-3-hydroxy-5-phenyl-pyrrolester durch Leukozyten in 0.1 M Trispuffer (pH 8.4) in Gegenwart von Aktivatoren und Detergentien. (Bestimmung der Umsetzungsgeschwindigkeit bei 330 nm).

Tabelle 3

| Molarität (NaCl) | Aktivator (125 µg/Test) | Detergens | µg/Test | relative Spaltungsgeschwindigkeit |
|---|---|---|---|---|
| - | n-Decanol | SDS | 10 | 1 |
| 0.1 | " | " | " | 6.35 |
| 0.2 | " | " | " | 13.0 |
| 0.25 | " | " | " | 17.4 |
| 0.3 | " | " | " | 14.6 |
| 0.4 | " | " | " | 7.7 |
| 0.5 | " | " | " | 7.2 |
| 0.25 | - | - | - | 3.2 |
| " | n-Decanol | - | - | 4.6 |
| " | " | SDS | 10 | 16.6 |
| " | n-Undecanol | - | - | 10.3 |
| " | " | SDS | 10 | 22.8 |
| " | " | BDTA | 20 | 5.1 |
| " | " | Heparinoid | " | 5.2 |
| " | " | Poly-DL-Lys | " | 8.4 |
| " | n-Dodecanol | SDS | 10 | 18.2 |
| " | " | BDTA | 20 | 4.2 |
| " | " | Heparinoid | " | 3.9 |
| " | n-Tridecanol | SDS | 10 | 15.0 |
| 0.5 | - | - | - | 4.3 |
| " | N-Undecanol | - | - | 10.4 |
| " | " | SDS | 10 | 16.4 |
| " | " | Heparinoid | 20 | 7.8 |
| " | n-Dodecanol | " | " | 6.0 |
| " | n-Tridecanol | " | 20 | 5.4 |

SDS = Natriumdodecylsulfat
BDTA = Benzyl-dimethyl-tetradecylamin-hydrochlorid

Tabelle 3a

| Salz | Molarität | Aktivator (125µg/ Test) | Detergens (10 µg/ Test) | relative Spaltungsgeschwindigkeit |
|---|---|---|---|---|
| - | - | n-Decanol | SDS | 1 |
| Na-Phosphat | 0,25 | n-Undecanol | " | 9.0 |
| $Na_2SO_4$ | " | " | " | 9.5 |
| KCl | " | " | " | 15.1 |
| $MgSO_4$ | " | " | " | 9.8 |

Beispiel 6

Einfluß der Natriumchloridkonzentration auf die Spaltung von N-Tosyl-L-alanin-3-hydroxy-5-phenylpyrrolester in Carbonatpuffer bei pH 8.4 durch Leukozyten in Gegenwart von unterschiedlichen Aktivatoren und Detergentien.
(Bestimmung der Spaltungsgeschwindigkeit bei 330 nm.)

Tabelle 4

| Puffer | NaCl (Mol) | Aktivator | Detergens | relative Spaltungsgeschwindigkeit |
|---|---|---|---|---|
| 0.1 M Tris | - | n-Decanol | SDS | 1 |
| 0.1 M Carbonat | - | n-Undecanol | SDS | 5.8 |
| | - | " | Hep. | 6.2 |
| | - | " | BDTA/SDS | 8.4 |
| | - | " | BDTA/Hep. | 6.8 |
| | 0.25 | " | SDS | 24.5 |
| | " | " | BDTA | 6.5 |
| | " | " | Hep. | 16.8 |
| | 0.25 | " | BDTA/SDS | 19.5 |
| | " | " | BDTA/Hep. | 20.1 |

SDS = Natriumdodecylsulfat (10 µg/Test)

BDTA = Benzyl-dimethyl-tetradecylamin-hydrochlorid (20 µg/Test)

Hep = Heparinoid (20 µg/Test)

Beispiel 8

Einfluß des Salzgehaltes auf die Spaltung von N-Tosyl-L-alanin-3-hydroxy-5-phenyl-pyrrolester in 0.1 M Boratpuffer pH 8.4 durch Leukozyten in Gegenwart von Undecanol als Beschleuniger und SDS als Detergenz
(Bestimmung der Spaltungsgeschwindigkeit bei 320 nm).

Tabelle 5

| Salz | Molarität | relat. Spaltungsgeschwindigkeit |
|---|---|---|
| LiBr | 0.1 | 1.5 |
| NaBr | " | 1.8 |
| KBr | " | 1.1 |
| LiCl | 0.25 | 1.55 |
| NaCl | " | 1.85 |
| KCl | " | 2.05 |
| $NH_4Cl$ | 0.1 | 1.4 |
| NaF | " | 1.15 |
| KF | " | 1.5 |
| $Na_2SO_4$ | 0.05 | 1.9 |
| " | 0.1 | 2.3 |
| " | 0.15 | 2.4 |
| " | 0.2 | 2.8 |
| " | 0.25 | 2.6 |
| " | 0.3 | 2.1 |
| $K_2SO_4$ | 0.1 | 2.05 |
| " | 0.15 | 2.35 |
| " | 0.2 | 2.05 |
| " | 0.25 | 1.1 |
| Natriumsuccinat | 0.1 | 1.15 |

Allgemeine Arbeitsvorschrift zur Herstellung der N-Tosyl-L-alanylester:

Die Ester wurden jeweils durch Umsetzung von N-Tosyl-L-alanylchlorid mit den Phenolen in absolutem Methylethylketon oder absolutem Toluol in Gegenwart von gepulvertem Kaliumcarbonat hergestellt. Nach 6 bis 12-stündigem Rühren bei ca. 55 °C waren zwischen 40 und 70 % des Phenols umgesetzt. Das Molverhältnis Phenol: $K_2CO_3$: Säurechlorid betrug zumeist 1:1,5:1,5. Der pH-Wert lag während der gesamten Reaktionszeit um 7. Zur Aufarbeitung wurde das Kaliumcarbonat bei 50 °C abfiltriert und danach das Lösungsmittel im Vakuum abdestilliert. Die Reinigung erfolgte über Säulenchromatographie mit Kieselgel (Laufmittel Petrolether: Aceton = ca. 9:1) und anschließendes Umkristallisieren.

L-p-Tosylalanin

Literatur: E. Fischer u. W. Lipschitz, B. 48, 362 (1915).

83,7 g (0,93 Mol) L-Alanin werden in 465 ml ca. 2N-Natronlauge gelöst. Die Lösung wird bei 70-72 °C portionsweise mit 186 g (0,976 Mol) p-Toluolsulfochlorid in 20 Minuten versetzt. Während der Zugabe des Sulfochlorids wird die Reaktionsmischung durch einen automatischen Titrator mit ca. 2N-Natronlauge bei pH

10 gehalten; dabei werden 560 ml 2N-Natronlauge verbraucht. Wenn sich der pH der Reaktionsmischung nicht mehr ändert, kühlt man diese auf 15-5 °C ab und stellt mit 37 %iger Salzsäure auf pH 3 ein. Das abgeschiedene Produkt wird abgesaugt, den feuchten Filterkuchen löst man aus 2350 ml Wasser um.
Ausbeute: 185,5 g (82 % der Theorie) L-p-Tosylalanin vom m.p. 132-135 °C.

p-Tosyl-L-alanylchlorid

158,1 g (0,65 Mol) L-p-Tosylalanin werden in 350 ml Thionylchlorid bei 40 °C gerührt, bis eine klare Lösung entstanden ist. Dann destilliert man das überschüssige Thionylchlorid im Wasserstrahlvakuum ab. Den Kolbenrückstand nimmt man in 300 ml destilliertem Toluol auf. Man erhält eine klare, schwach gelbliche Lösung, die in 900 ml gerührtes Waschbenzin eingegossen wird. Das Säurechlorid fällt aus. Es wird am nächsten Tage abgesaugt, mit Leichtbenzin gewaschen und in einem Vakuumexsiccator über Calciumchlorid/Kaliumhydroxyd getrocknet.
Ausbeute: 155 g (91 % der Theorie) an fast farblosen Kristallen vom m.p. 81-83 °C.

## Patentansprüche

1. Mittel zum Nachweis esterolytischer und/oder proteolytischer Enzyme, enthaltend
   (a) einen Aminosäure- oder Peptidester eines Phenols als chromogenes Enzymsubstrat und
   (b) 0,05 M bis 2 M einer die enzymatische Spaltung der Aminosäure- bzw. Peptidesterbindung von Komponente (a) beschleunigende Substanz,
   dadurch gekennzeichnet, daß die beschleunigende Substanz ein Salz eines Kations der Gruppe $Li^+$, $Na^+$, $K^+$ oder $Mg^{++}$ und eines Anions der Gruppe Halogenid, Pseudohalogenid, Sulfat, Phosphat, Acetat, Trifluoracetat, Toluolsulfonat oder Succinat ist.

2. Mittel nach Anspruch 1, dadurch gekennzeichnet, daß es als zusätzliche beschleunigende Substanz eine Polyaminosäure mit einem Molekulargewicht zwischen 1000 und 2 000 000, vorzugsweise zwischen 500 und 500 000, enthält.

3. Mittel nach Anspruch 2, dadurch gekennzeichnet, daß die Polyaminosäure aus nur einer Aminosäure, aus zwei oder mehreren verschiedenen Aminosäuren mit statistischer Sequenz oder aus zwei oder mehreren verschiedenen Aminosäuren mit sich wiederholender Aminosäuresequenz aufgebaut ist.

4. Mittel nach Anspruch 2 oder 3, dadurch gekennzeichnet, daß die Polyaminosäure aus Aminosäuren der allgemeinen Formel

   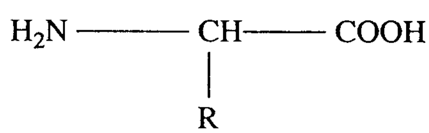

   aufgebaut ist, in welcher R für Wasserstoff oder einen gegebenenfalls verzweigten Alkyl-, Cycloalkyl- oder Aralkylrest mit 1 bis 15 C-Atomen, bevorzugt 1 bis 9 C-Atomen, steht, welcher gegebenenfalls durch eine oder 2-Hydroxy-, Mercapto-, Carboxyl-, Amino oder Guanidinogruppen substituiert ist.

5. Mittel nach Anspruch 2 bis 4, dadurch gekennzeichnet, daß 5 bis 100 Mol-%, bevorzugt 10 bis 100 Mol-%, der Aminosäurebausteine der Polyaminosäure eine basische Gruppe tragen.

6. Mittel nach Anspruch 1 bis 5, dadurch gekennzeichnet, daß die Reagenzien in einem inerten Trägermaterial, vorzugsweise in Form eines Teststreifens, inkorporiert sind.

7. Verfahren zum Nachweis von esterolytischen und/oder proteolytischen Enzymen in flüssigen Proben, insbesondere Körperflüssigkeiten, dadurch gekennzeichnet, daß man die Probe mit einem Mittel nach Anspruch 1 bis 6 in Kontakt bringt und die auftretende Farbreaktion bestimmt.

## Claims

**1.** Agent for the detection of esterolytic and/or proteolytic enzymes, containing
(a) an amino acid ester or peptide ester of a phenol, as the chromogenic enzyme substrate, and
(b) 0.05 M to 2 M of a substance which accelerates the enzymatic cleavage of the amino acid ester or peptide ester bond of component (a),
characterised in that the accelerating substance is a salt of a cation of the group consisting of $Li^+$, $Na^+$, $K^+$ and $Mg^{++}$ and of an anion of the group consisting of halide, pseudohalide, sulphate, phosphate, acetate, trifluoroacetate, toluenesulphonate and succinate.

**2.** Agent according to Claim 1, characterised in that it contains a polyamino acid with a molecular weight of between 1,000 and 2,000,000, preferably between 500 and 500,000, as an additional accelerating substance.

**3.** Agent according to Claim 2, characterised in that the polyamino acid is built up from only one amino acid, from two or more different amino acids in random sequence, or from two or more different amino acids with a recurring amino acid sequence.

**4.** Agent according to Claim 2 or 3, characterised in that the polyamino acid is built up from amino acids of the general formula

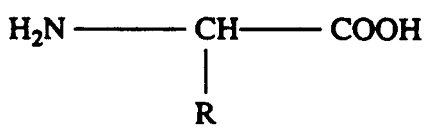

in which R represents hydrogen or an optionally branched alkyl, cycloalkyl or aralkyl radical which has 1 to 15 carbon atoms, preferably 1 to 9 carbon atoms, and is optionally substituted by one or 2 hydroxyl, mercapto, carboxyl, amino or guanidino groups.

**5.** Agent according to Claims 2 to 4, characterised in that 5 to 100 mol%, preferably 10 to 100 mol%, of the amino acid units of the polyamino acid carry a basic group.

**6.** Agent according to Claims 1 to 5,characterised in that the reagents are incorporated in an inert carrier, preferably in the form of a test strip.

**7.** Process for the detection of esterolytic and/or proteolytic enzymes in liquid samples, in particular body fluids, characterised in that the sample is brought into contact with an agent according to Claims 1 to 6 and the colour reaction which occurs is determined.

**Revendications**

**1.** Moyen de détection d'enzymes estérolytiques et/ou protéolytiques contenant
a) un ester d'acide aminé ou de peptide d'un phénol comme substrat chromogène des enzymes et
b) 0,05 M à 2 M d'une substance accélérant la scission enzymatique de la liaison de l'ester d'acide aminé ou de peptide de l'élément a),
caractérisé en ce que la substance accélératrice est un sel d'un cation du groupe $Li^+$, $Na^+$, $K^+$ ou $Mg^{++}$ et d'un anion du groupe des halogénures, pseudohalogénures, sulfates, phosphates, acétates, trifluoroacétates, toluènesulfonates ou succinates.

**2.** Moyen selon la revendication 1, caractérisé en ce qu'il contient comme substance accélératrice supplémentaire un polypeptide avec un poids moléculaire entre 1.000 et 2.000.000, de préférence entre 500 et 500.000.

**3.** Moyen selon la revendication 2, caractérisé en ce que le polypeptide se compose d'un seul acide aminé, de deux ou plusieurs acides aminés différents avec une séquence statistique ou de deux ou plusieurs acides aminés différents avec une séquence d'acides aminés répétitive.

**4.** Moyen selon la revendication 2 ou 3, caractérisé en ce que le polypeptide se compose d'acides

aminés de formule générale

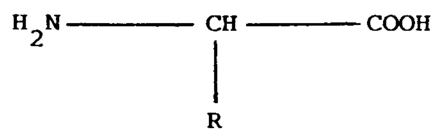

dans laquelle R désigne de l'hydrogène ou un radical alkyle, cycloalkyle ou aralkyle éventuellement ramifié avec 1 à 15 atomes C, de préférence 1 à 9 atomes C, qui est éventuellement substitué par un ou deux radicaux hydroxy, mercapto, carboxyle, amino ou guanidino.

**5.** Moyen selon les revendications 2 à 4, caractérisé en ce que 5 à 100 % en mole, de préférence 10 à 100 % en mole des éléments constitutifs des acides aminés des polypeptides portent une fonction basique.

**6.** Moyen selon les revendications 1 à 5, caractérisé en ce que les réactifs sont intégrés dans un matériau de support inerte, de préférence sous forme de bande de test.

**7.** Procédé de détection d'enzymes estérolytiques et/ou protéolytiques dans des échantillons liquides, en particulier dans des liquides corporels, caractérisé en ce qu'on met l'échantillon en contact avec un moyen selon les revendications 1 à 6 et que l'on détermine la réaction de coloration qui se produit.